# EUROPEAN PATENT SPECIFICATION

(11) **EP 3 486 655 B1**
(45) Date of publication and mention of the grant of the patent: **20.05.2020**
(21) Application number: 18198572.2
(22) Date of filing: 04.10.2018
(51) Int. Cl.: G01N 33/543, G01N 33/483

(54) **METHOD FOR IMMUNOCHEMICAL ASSAYS ON DRIED BLOOD**
VERFAHREN ZUR IMMUNOCHEMISCHEN UNTERSUCHUNG VON GETROCKNETEM BLUT
MÉTHODE POUR DES DOSAGES IMMUNOCHIMIQUES DE SANG SÉCHÉ

(30) Priority: 16.11.2017 IT 201700131165
(43) Date of publication of application: 22.05.2019
(73) Proprietor: Humanbiocare S.R.L., 47838 Riccione (RN) (IT)
(72) Inventor: VOLPI, Nicola, 41125 Modena (MO) (IT); MANTOVANI, Veronica, 42047 Rolo (RE) (IT); GALEOTTI, Fabio, 41038 San Felice sul Panaro (MO) (IT); NERI, Enrico, 47843 Misano Adriatico (RN) (IT)
(74) Representative: Manfredi, Irene

(56) References cited:
- EP-A1- 2 529 769
- WO-A2-2005/050224
- US-B1- 6 309 887
- XIANG YUHONG ET AL: "Adapting dried blood spot sampling for an anti-therapeutic antibody immunogenicity assay", JOURNAL OF IMMUNOLOGICAL METHODS, ELSEVIER SCIENCE PUBLISHERS B.V.,AMSTERDAM, NL, vol. 393, no. 1, 18 April 2013 (2013-04-18), pages 53-60, XP028541467, ISSN: 0022-1759, DOI: 10.1016/J.JIM.2013.04.006
- BELLISARIO R ET AL: "Simultaneous measurement of antibodies to three HIV-1 antigens in newborn dried blood-spot specimens using a multiplexed microsphere-based immunoassay", EARLY HUMAN DEVELOPMENT, SHANNON, IR, vol. 64, no. 1, 1 August 2001 (2001-08-01) , pages 21-25, XP002421127, ISSN: 0378-3782, DOI: 10.1016/S0378-3782(01)00167-0
- FABIO FORMENTI ET AL: "Comparison of S. stercoralis Serology Performed on Dried Blood Spots and on Conventional Serum Samples", FRONTIERS IN MICROBIOLOGY, vol. 7, 8 November 2016 (2016-11-08), XP055461582, DOI: 10.3389/fmicb.2016.01778
- ADAM H METHEREL ET AL: "Cryopreservation Prevents Iron-Initiated Highly Unsaturated Fatty Acid Loss during Storage of Human Blood on Chromatography Paper at -20°C", THE JOURNAL OF NUTRITION, vol. 145, no. 3, 7 January 2015 (2015-01-07), pages 654-660, XP055461816, US ISSN: 0022-3166, DOI: 10.3945/jn.114.203679

## Description

The present invention is directed to a method for performing immunochemical analyses on dried blood samples, to a device for collecting samples of blood to be dried, obtainable from said method, and to its use in immunochemical assays, to a kit for performing immunochemical analyses on dried blood samples comprising said device, and to its use in intolerance or allergy diagnostic methods.

Immunochemical assays are commonly used to determine the presence and/or the concentration of (total or specific) antibodies directed against antigens and/or allergens in serum. The present invention allows to perform immunochemical analyses that detect the presence of antibodies directed against antigens and/or allergens, in dried blood samples, and to *in vitro* diagnose intolerances and/or allergies, for instance the food borne ones. Preferably, such analyses are performed by using the microarray technology.

Common immunochemical assays include: the enzyme linked immunosorbent assay (ELISA), the radio immuno assay (RIA), the fluorescent immuno assay (FIA), and the chemiluminescent assay (CLA). Typically, in order to assess the presence of specific antibodies in a subject's blood, serum prepared from fresh blood drawn from such subject is incubated with antigens consisting of recombinant or natural peptides, which are extracted, purified and immobilized on a solid phase; incubation takes place under standardized conditions, such as to allow a chemical bonding between the antibodies present in the serum and the antigenic peptides, which results in formation of antigen-antibody complexes; the antibody-antigen complexes are detected, after one or more washings aimed at eliminating the not bound antibodies, by using any detection means known in the art, for instance by using a reagent comprising a secondary antibody that is fluorescent or that is linked to an enzyme detectable by way of chemiluminescence, said secondary antibody binding to the first antibody and consequently to the antigen-antibody complex.

High-throughput immunochemical analyses on small volumes of samples can be performed by using a protein microarray technology, wherein proteins are immobilized on solid substrates having micrometric dimensions (high density matrices, also called chips or biochips).

EP0981050 describes an ELISA method for detecting allergens from hazelnuts; WO2002029415 describes a method for a qualitative assessment of IgG and IgE in serum by way of microarray technology; US20030073249 and US20060008895 describe the preparation of microarray chips for assessing IgE/IgG in a sample. However, none of said documents discloses immunochemical analyses on dried blood, i.e. on blood adsorbed on a solid support and let, at least partially, dry in the air before being analyzed.

Xiang Yuhong et al (2013) relates to the detection of anti-drug antibodies using a support for dried blood; Bellisario R et al (2001) relates to the detection of HIV-1 antibodies in dried blood spot samples; Fabio Formenti et al (2016) discloses detection of antibodies in supported dried blood samples in different conditions; WO 2005/050224 discloses the recovery of antibodies from dried blood samples on different filter papers. None od said documents discloses the treatment of blood samples supports with glycerol.

Adam H Metherel et al (2015) discloses the pretreatment of filter paper with 40% glycerol, but it does not relate to the detection of antibodies.

EP2529769 discloses a hollow-fiber membrane treated with 83.5% or 91.2% glycerol, however for use in blood purification methods by filtration and not for collecting a blood sample.

The dried blood samples offer a number of advantages with respect to serum or plasma samples separated from fresh blood as conventionally used in immunochemical analyses. As a matter of fact, dried blood samples reduce the operating complexity of analysis, do not need serum or plasma to be separated, are less invasive, making it possible to use very small volumes of blood, are easy to store and transport, minimize the risk of infections for the operators who handle them. Therefore, their use for immunochemical analyses, especially for diagnostic purposes, is highly desired.

Dried blood samples have been used for immunoenzymatic analyses on microarrays, for assessing immunoglobulins directed against cytokines (Jiang W et al., J Immunol Methods 403, 79-86, 2014) or against proteins associated with pregnancy (Pennings JL et al., Dis Markers 509821, 2014, 2014). However, in order to perform such analyses, dried blood samples had to be analyzed in short times, within 24 hours as from blood draw, being stored at -80°C, otherwise the immunoglobulins contained in dried blood would rapidly degrade. In particular, as demonstrated hereafter, it is not possible to carry out immunochemical analyses in solid phase, for instance by using a microarray technology, starting from dried blood samples and optically assessing the antigen-antibody complexes, because of formation of haloes on the solid substrate that comprises the immobilized antigens. Without wishing to be bound to theory, such haloes might be due to unspecific binding of antibodies or of other proteins and biomolecules present in the dried blood sample on a non-pre-treated support, that are degraded or that feature an altered reactivity, to the antigens being immobilized on the substrate.

Therefore, the use of dried blood for immunochemical analyses features, as at today, a number of limitations and therefore it did not become widespread.

The present invention solves the technical problem of performing immunochemical analyses on dried blood samples, by using any immunochemical assays known in the art, including large scale analyses by way of a microarray technology, while maintaining the immunological response active, i.e. keeping the antibodies contained in the dried blood samples biologically active for much longer times as compared to what experimented in the art, and allowing an efficient conservation of samples even at room temperature.

In particular, the present invention is directed to a method for carrying out immunochemical analyses on dried blood samples, characterized by having a step whereby the supports on which the drawn blood samples will be adsorbed and dried, are pre-treated: in said pre-treatment step, supports are soaked, saturated, with a solution comprising glycerol, in a percentage concentration of at least 70% by volume (v/v), as described in details below; the pre-treated supports are stored, preferably in the air, at room temperature and for a time period less than or equal to three months, prior to receiving the blood sample. Surprisingly glycerol allows to keep the antibodies (immunoglobulins), that are present in the blood adsorbed by the support, biologically active even after drying, for long periods of time, and to store the samples at room temperature or at lower temperatures.

Preferably, the method according to the present invention comprises eluting a sample from a pre-treated support, incubating the eluted blood sample with one or more antigens, under conditions suitable to form antigen-antibody complexes, and detecting said complexes by using any conventional method, known to those skilled in the art (for instance, by way of a physical, enzymatic, chemical, etc., reaction).

The term antigen indicates any molecule or fragment of molecules capable of being recognized and be bound by an antibody; typically, an antigen is a peptide, a polypeptide, a protein, and fragments thereof.

The dried samples, eluted from the pre-treated supports according to the invention and analyzed in immunochemical assays, exhibit maintenance of the immunological response, as assessed in terms of formation of specific antigen-antibody complexes. Also, the pre-treatment does not interfere with antibody-antigen complex optical detection systems in the immunochemical assay: as a matter of fact, unlike the dried blood samples eluted from non-pre-treated supports, the dried blood samples eluted from pre-treated supports according to the method of this invention do not comprise any substances interfering with the antigen-antibody complex detection systems.

The present invention is also directed to a device for collecting and drying a blood sample, comprising the pre-treated support as described above, and to a kit for detecting and/or measuring antibodies directed against specific antigens in dried blood samples, said kit comprising the support treated according to the method of this invention, a solid substrate comprising at least one surface on which one or more antigens are immobilized, and a reagent suitable for detecting the antigen-antibody complexes comprising the antibodies bound to the one or more antigens. Preferably said one or more antigens are food antigens, i.e. antigens extracted from foods.

Finally, the invention is directed to the use of said pre-treated support in immunochemical assays and of said kit in an *in vitro* diagnostic method, preferably for food allergies and/or intolerances, on blood samples drawn from human or animal subjects, in which method the presence of antibody-antigen complexes indicates the presence of an intolerance/allergy to said antigen; for instance, in the case of food antigens, the assessment of antibody-antigen complexes can indicate the presence of an intolerance/allergy to the food from which said antigen is extracted.

Further characteristics and advantages of the invention will be apparent from the following detailed description of preferred but not exclusive embodiments of the invention and from the enclosed figures, wherein:

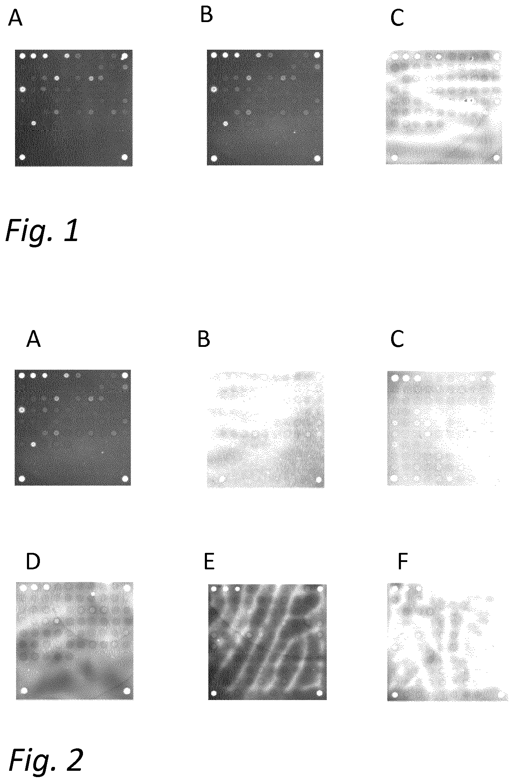

figure 1 shows antigen-antibody complexes (light dots) detected on three surfaces (PADs) of a microarray chip, incubated respectively with: A) a sample of plasma separated from fresh blood; B) a dried blood sample, eluted from a pre-treated paper support (DBS), according to a preferred embodiment of the invention; C) a dried blood sample eluted from a non-pre-treated paper support (DBS). All the blood samples are drawn from one and the same subject;
figure 2 shows detection of antigen-antibody complexes on six PADs of a microarray chip incubated with dried blood samples, eluted from DBS supports pre-treated with: A) a 95% (by volume) glycerol solution in water; B) a hyaluronic acid solution (5 mg/ml); C) a poloxamer solution (10 mg/ml); D) a dextran solution (10 mg/ml); E) a polystyrene solution (2 mg/ml); F) an elastomeric polymer solution (1 mg/ml);

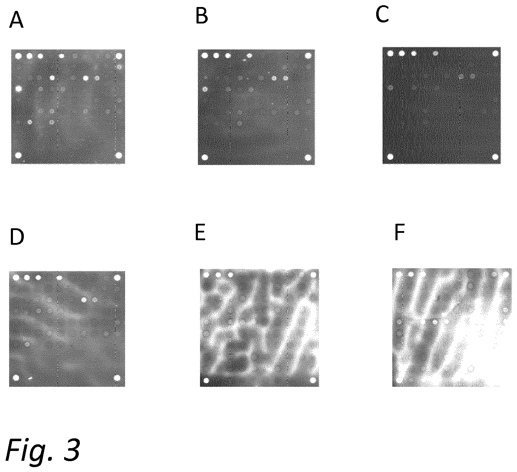

figure 3 shows detection of antigen-antibody complexes on six PADs of a microarray chip incubated with blood samples dried and eluted from DBS supports treated with a glycerol solution in water, wherein the glycerol:water volume ratio is respectively: A) 100:0; B) 90:10; C) 80:20; D) 70:30; E) 60:40; F) 50:50;

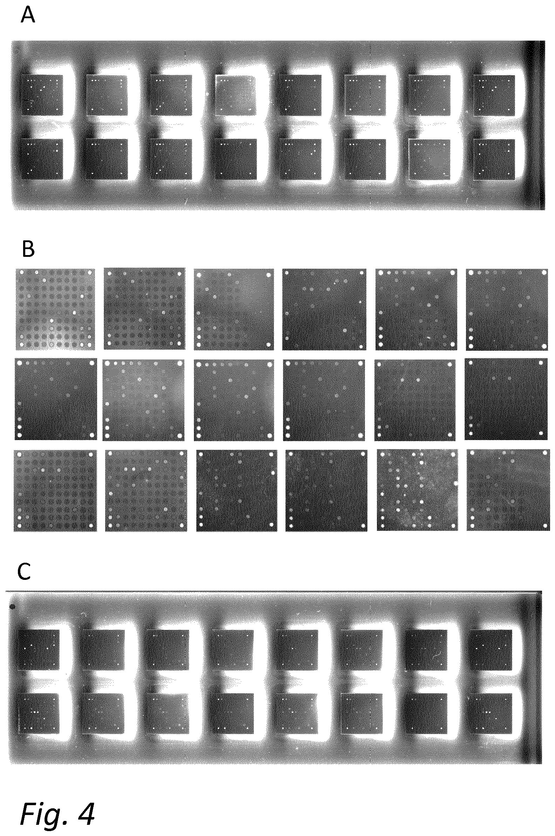

figure 4 shows detection of antigen-antibody complexes on 16 PADs of one and the same (A, C) or of different (B) microarray chips, on which the antigens are immobilized, incubated with blood dried on a support pre-treated with the glycerol solution, according to a preferred embodiment of the invention, and wherein the device comprising the pre-treated support is: A) a paper DBS, B) a cellulose plug, C) a cotton-bud;

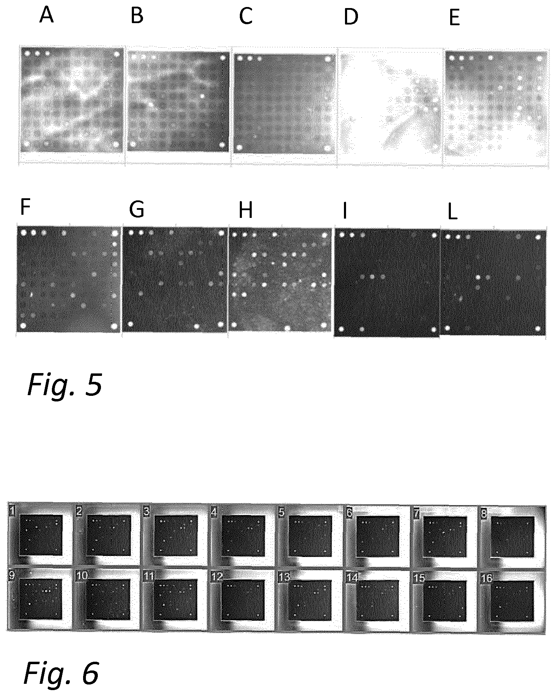

figure 5 shows detection of antigen-IgG complexes on PADs of different microarray chips, incubated with dried blood samples, eluted from non-pre-treated DBSs (A-E) or pre-treated DBSs(F-L), analyzed after 2 days (A, F), 7 days (B, G), 15 days (C, H), 21 days (D, I), or 30 days (E, L) after blood deposition on the support;
figure 6 shows detection of IgE-antigen complexes on PADs of a microarray chip, incubated with different dried blood samples, eluted from pre-treated supports according to the invention.

The device of the invention for collecting and drying a blood sample comprises a support capable of adsorbing a liquid, preferably comprising polymer fibers, more preferably cellulose fibers, either natural or derived from natural or artificial fibers (synthetic fibers). The support is preferably a paper or cotton one. Preferred devices include: devices comprising paper supports, also called dried blood spots or DBS, marketed under the tradename DBS Whatman 903™, and devices comprising cotton substrates, such as cotton-buds or those used for blood drawing purposes.

The glycerol solution, with which the substrates are soaked in the pre-treatment step according to the invention, comprises at least about 70% of glycerol by volume and optionally water. Preferably, the glycerol:water volume ratio ranges from 70:30 to 100:0, more preferably from 80:20 to 90:10. Preferably, the glycerol solution comprises glycerol at a percentage concentration greater than or equal to about 75% by volume, greater than or equal to about 80%, greater than or equal to about 90%, greater than or equal to about 95%, equal to about 100% by volume.

Preferably, the supports pre-treated with the glycerol solution are stored at room temperature (equal to about 25°C) for a period of time shorter than or equal to 3 months, more preferably for a period of time ranging from one hour to 3 months, before depositing a blood sample thereon.

The blood samples adsorbed on the pre-treated supports according to the invention are preferably analyzed in a time interval ranging from 1 day to 1 month after being laid down on the pre-treated support, being stored at room temperature. The blood sample adsorbed on the pre-treated support can be let in the air at room temperature for one month and be still effectively used in immunochemical assays.

The method according to the present invention comprises analyzing the dried blood sample by way of any immunochemical assay known in the art; preferably, said assay is suitable for detecting and/or quantifying the presence of antibodies or fragments thereof binding the antigen in the dried blood sample; more preferably said antibodies are immunoglobulins selected in the group comprising: immunoglobulins G (IgG), immunoglobulins E (IgE), immunoglobulins M (IgM) or immunoglobulins A (IgA), including their specific sub-classes and their antigen binding fragments (Fabs). Preferably said antibodies are immunoglobulins directed against food antigens or allergens; more preferably said immunoglobulins are IgG immunoglobulins, even more preferably of the IgG4 sub-class, and/or IgEs and/or IgAs immunoglobulins.

It is noted that the presence in a blood sample of IgEs, IgGs and/or IgAs specific against food antigens is often associated with food allergies or intolerances in the subject from whom the sample is drawn. Generally, the presence of IgGs specific against food antigens is associated with intolerances to foods that contain such antigens, whereas the presence of IgEs specific against food antigens is associated with allergies to foods that contain such antigens. The present invention can thus be used for the *in vitro* diagnosis of food born intolerances and/or allergies. Further, the present invention can be used in the diagnosis of immunodeficiencies, autoimmunity, candidiasis, microflora imbalance, and dysfunctions of the intestinal mucosa.

The dried blood sample to be analyzed by way of an immunochemical assay is eluted from the pre-treated support in a solution suitable for performing the immunochemical assay. A suitable solution is, for exemplary non-limitative purposes only, a Tris-HCl 50 mM, NaCl 150 Mm solution, having a pH of 7.2, as apparent to those skilled in the art.

Preferably the immunochemical assay of the method according to the present invention comprises incubating the blood sample with one or more antigens immobilized on a surface of a solid substrate. The term immobilization refers to the linkage or fixation of antigens to a surface of a solid substrate by using conventional means; for instance, covalent bonds might form between residues of the molecules to be immobilized and reactive groups of the material that makes up the surface of the substrate; this link is either a direct link between the antigen and the substrate, or indirect, the antigen being linked to the substrate via a specific antibody which is in turn directly immobilized on a surface of the support.

Preferably the immunochemical assay of the method according to the present invention is an ELISA assay, more preferably an assay wherein the antigens are immobilized on a microtiter plate.

Preferably, the immunochemical assay of the method according to the present invention is performed by using the microarray technology, wherein the one or more antigens are immobilized on a microarray chip. The chip can have any shape be made from any material; preferably the chip comprises one or more reactive surfaces distributed on the surface area of the chip and on which the one or more antigens are immobilized; for reactive surfaces it is meant surfaces made from materials comprising reactive chemical groups capable of linking proteins and peptides, for instance aldehydic or amino groups. Preferably the chip is made from glass, plastic, or metal, and comprises one or more reactive surfaces made by nitrocellulose, silicon dioxide, an epoxy material, or other materials comprising reactive groups capable of linking proteins and peptides. Preferably, the microarray chip comprises a glass substrate, comprising a number of reactive surfaces (PADs) in the range from 2 to 200, for instance comprising 2, 4, 8, 16, or 24 PADs, on which one or more antigens are immobilized. More preferably, the microarray chip is a slide having dimensions of about 25x76 mm, comprising 8 or 16 PADs; preferably every PAD is a 6x6 mm square area; more preferably said PADs are made from nitrocellulose. Every chip possibly comprises 50 to 350 antigens/cm²; preferably every PAD comprises up to 200 antigenic peptides.

The use of the microarray methodology makes it possible to dose specific immunoglobulins, directed to up to 200 different allergens, in a volume of dried blood sample of about 1 µL, in periods of time ranging from 2 to 4 hours.

Preferably said one or more antigens are extracted and purified peptides or proteins, be they natural or recombinant; purification of said antigens can take place by way of gel-chromatography, for instance by way of size-exclusion chromatography (SEC) or by using hydrophobic resins, which operation is followed by an ultracentrifugation at 50,000 x g for 30 minutes and an ultrafiltration through a 0.22 µm filter; preferably, the purified extracts are dosed by way of any colorimetric assay known in the art and tested for their quality on polyacrylamide gel electrophoresis.

Preferably, the extracted and purified antigens are immobilized on a solid substrate by using methods known in the art, for instance they can be put in an NaHCO₃ 20 mM solution at a concentration of 10 µg/ml and linked to the reactive surfaces of the substrate in the presence of a relative humidity ranging from 30% to 50% and a temperature of 25°C.

Said one or more antigens can be immobilized on a substrate, individually or mixed.

Preferably said one or more antigens are extracted from one or more foods; said foods include without limitations: milk, eggs, yeast, meat, fish, clams, crustaceans, cereals, seeds, hazelnuts, bouillon cubes, vegetables, fruits, chocolate, spices, and derivatives thereof.

Antigen-antibody complexes, which form after incubating a blood sample with one or more antigens, are assessed in an immunochemical assay by using any conventional method that is known to those skilled in the art. For instance, after incubation with one or more antigens of the blood sample, eluted from the pre-treated supports according to the present invention, incubation is stopped and the surface of the substrate is washed one or more times in order to remove any unspecific complexes formed, then a reagent is added, for instance a secondary antibody, capable of recognizing and specifically interacting with an antibody of interest and provided with a molecule capable of reacting with a substrate or of fluorescing if energized, and generating a signal which can be optically assessed. Preferably said molecule is selected from the group comprising: horseradish peroxidase (HRP) enzyme, hydroperoxidases, alkaline phosphorylase, beta-galactosidase, tyramide Alexa Flour 647, tyramide Alexa Flour 546, tyramide Alexa Flour 532, tyramide Cy3, or tyramide Cy5.

The signal generated by said molecule is analyzed, preferably by using a densitometric software, capable of quantifying the quantity of immunoglobulins bound to the antigens, by interpolating the intensity of the light signal, assessed in correspondence with the one or more antigens, on a calibration curve, built up by measuring the light intensity of the signal generated by antibodies at known, growing concentrations.

Without wishing to limit the invention, a preferred embodiment of the method according to the present invention is described below.

A blood sample is adsorbed and dried on a support pre-treated with a solution including glycerol and optionally water in a volume ratio of glycerol to water ranging from 70:30 to 100:0; the dried sample is eluted by putting said support in a Tris-HCl 50 mM, NaCl 150 mM solution, at a pH of 7.2, stirred for 15-30 minutes at room temperature, then centrifuging at 10,000 x g the solution per 10 minutes at 4°C and drawing the supernatant off. 20 µL of the supernatant are diluted by adding 980 µL of the elution buffer. A microarray chip comprising 1 to 16 PADs, preferably 16 PADs, on each of which up to 200 different antigens, preferably 92 different antigens, are immobilized, is washed with 5-10 ml of a Tris-HCl 50 mM, NaCl 150 mM washing buffer having a pH of 7.2 and comprising a detergent such as triton X-100, SDS or tween 20, preferably tween 20 at 0.05% by weight, for about 1 minute at room temperature. After removing the washing buffer, a blocking buffer containing bovine serum albumin (BSA) is added to saturate the unspecific chemical groups on the surface of the PADs, preferably by using 5 ml of tris-HCl 50 mM, NaCl 150 mM buffer at a pH of 7.2, comprising 0.05% by weight of tween 20 and 1% by weight of BSA, at room temperature for 30 minutes. After removing the blocking buffer and washing the chip three times with the washing buffer, 100 µL of the eluted sample are put in contact with each PAD under conditions suitable to enable the immunoglobulins present in the blood sample to react with the antigens immobilized on the PADs, at room temperature for 30 minutes. Every PAD is preferably separated from the other PADs by tight cells in order to prevent any cross contaminations between the different blood samples. After removing the non-reactive immunoglobulins and washing the chip three times with the washing buffer, 5 ml of a secondary antibody capable of specifically interacting with the immunoglobulins of interest and provided with a molecule capable of reacting with a substrate and generating a signal are added and incubated at room temperature for 30 minutes; preferably said secondary antibody is a human anti-IgG or anti-IgE monoclonal antibody, linked to a peroxidase enzyme. After removing the non-reactive secondary antibody and washing the chip three times with the washing buffer, 800 µL of a solution containing the peroxidase substrate, 3,3',5,5'-tetrametilbenzidine (TMB), are added to the chip and the reaction is let take place for about 10 minutes. After removing the TMB, the chip is washed out three times by using 5 ml of distilled water for 1 minute, centrifugated for 30 seconds in a dedicated microcentrifuge, then it is scanned and every PAD is analyzed by a densitometric analysis to determine the presence of antigen-antibody complexes and to extrapolate the concentration of immunoglobulins that are reactive to the antigens present in the dried blood sample. Preferably, growing amounts of immunoglobulins, for instance 9, 5, 10, and 20 picograms, are immobilized on the chip to get a calibration curve and quantify the immunoglobulins present in the blood sample, and the signal generated by them is plotted in the calibration curve. Then the detected signal for antigen-antibody complexes is interpolated in the calibration curve, thus tracing the picograms of antibodies linked to the antigens for every analyzed sample. Preferably one calibration curve is set for each PAD.

### EXAMPLE 1

In an immunochemical assay by way of the microarray methodology, a comparison is made of the biological activity of antibodies comprised in: a sample of plasma separated from fresh blood (Figure 1A), a sample of blood dried on a DBS support pre-treated with a 100% (by volume) glycerol solution and stored at room temperature for three months (Figure 1B), or a sample of blood dried on a non-pre-treated DBS support (Figure 1C), all drawn from one and the same subject. The activity of the antibodies is determined, in accordance with the above described procedure, on a glass microarray chip provided with 16 nitrocellulose PADs, on which food antigens are immobilized, with a density of 100 dots per PAD.

As shown in Figure 1, when optically assessed, the PAD incubated with blood dried on a non-pre-treated DBS exhibits numerous haloes due to an interfering material, which prevents a correct antibody to antigenic proteins reaction and makes the PADs not analyzable. Conversely, the blood dried on a DBS pre-treated according to the method of the present invention is found to be biologically active as much as fresh plasma. Note that such activity is present in dried blood samples according to the invention even after 30 days after blood deposition onto the pre-treated support. The same results have been achieved with blood samples dried on other supports (swabs, cotton-buds, supports made from cellulose or its derivatives, or artificial fibers) pre-treated according to the method of the invention.

### EXAMPLE 2

In an immunochemical assay, carried out with the microarray methodology as in example 1, the activity has been assessed of immunoglobulins present in dried blood samples adsorbed on supports pre-treated with different solutions: a solution comprising glycerol and water (in a ratio equal to 95:5 by volume), a solution comprising hyaluronic acid, or solutions comprising artificial polymers, such as poloxamer, dextran, polystyrene, or elastomeric polymers.

The blood dried on DBSs treated with hyaluronic acid or artificial polymer solutions (Figure 2B-F) leads to formation of haloes on the microarray chip, which prevent the possibility of assessing the antigen-antibody complexes present in the samples. Conversely, the blood extracted from the DBS pre-treated according to the method of the present invention (Figure 2A) exhibits an optically assessable antibody reactivity (light dots in Figure 2A). The same results have been achieved with blood samples dried on other substrates (swabs, cotton-buds, substrates made from cellulose or its derivatives, or artificial fibers) pre-treated according to the method of the invention.

### EXAMPLE 3

In an immunochemical assay, carried out according to the microarray methodology of example 1, the activity is assessed of antibodies comprised in blood samples dried on DBS support pre-treated with solutions comprising glycerol in different volumetric concentrations.

It is apparent from the tests that have been carried out that only the PADs incubated with blood dried on DBSs pre-treated with solutions comprising at least 70% of glycerol by volume show reactivity of the immunoglobulins with the immobilized antigens (light dots in figures 3A-D) and are processable via a densitometric analysis. The same results have been achieved with blood samples dried on other substrates (swabs, cotton-buds, substrates made from cellulose or its derivatives, or artificial fibers) pre-treated according to the method of the invention.

### EXAMPLE 4

Solutions are prepared comprising antigens extracted from 92 different foods and purified, in a concentration of 10 µg/ml of total proteins. These foods include: anchovies, garlic, apricot, pineapple, duck, peanuts, orange, asparagus, banana, basil, chard, see bass, cocoa, coffee, chamomile, artichoke, carrot, chestnut, horse, cauliflower, chickpeas, cucumber, cherry, onion, rabbit, French beans, emmer, beans, fennel, strawberry, edible boleti, crawfishes, durum wheat, bread wheat, kiwi, goat milk, cow's milk, sheep milk, lettuce, lentils, beer yeast, lemon, pork, maize, barley malt, tangerine, almond, beef meat, apple, aubergine, bilberry, olive, lobster, oregano, barley, Parmesan cheese, potato, black pepper, peppers, pear, peach, pine nuts, peas, chicken, tomato, parsley, plum, rice, rosemary, salmon, celery, cuttlefish, sole, soya bean, spinach, turkey, green tea, tuna fish, trout, eggs, white grapes, vanilla, clams, pumpkin, zucchini.

A glass microarray chip is provided, equipped with 16 nitrocellulose PADs, on which purified peptides are immobilized in the presence of a relative humidity of 35% and at room temperature, at a density of 100 dots per PAD (every dot corresponding to one peptide/protein). The chip is washed and blocked, as described above.

Blood samples adsorbed on DBSs pre-treated with an 85% (by volume) glycerol solution in water, stored at room temperature, are eluted, one month after deposition onto the support, in 1 ml of Tris-HCl 50 mM, NaCl 150 mM buffer at a pH of 7,2, under continuous stirring for 20 minutes at room temperature, then they are incubated with the antigens immobilized on the chip.

After incubation, the immunoglobulins not linked to antigens are washed away and a human anti-IgG monoclonal secondary antibody provided with peroxidase is made interacting with the IgGs bound to the immobilized antigens. After washing, the portion of the peroxidase that is present on the secondary antibody is reacted with its respective TMB substrate, to generate visible spots having different intensities (Figure 4A). A quantitative measurement of the visible products of the reaction is carried out by using a highly specific dedicated densitometric software: the concentration of the IgGs bound to the antigens present in the samples is determined by using, as a calibration curve, the signals provided by IgGs immobilized at known, growing concentrations on the chip.

### EXAMPLE 5

Solutions are prepared comprising extracted and purified, single or mixed antigens and allergens, at a concentration of 20 µg/ml of total proteins, from: cow's milk, sheep milk, goat milk, beer yeast, chemical yeast, emmer, barley, wheat, maize, rice, rabbit, pork, lamb, turkey, chicken, eggs, beef, salmon, tuna fish, mix of cephalopods (cuttlefishes, calamari, octopus), cod, sole, crawfishes, zucchini, pumpkin, turkey, chicken, eggs, beef meat, salmon, aubergine, peppers, chicory, lettuce, carrot, asparagus, spinach, tomatoes, mushrooms, potatoes, celery, onion, cauliflower, peach, grape, kiwi, orange, lemon, apricot, peas, soya beans, peanuts, almond, olive, cocoa, coffee, white sugar, honey.

The antigens are immobilized on 18 different glass microarray chips by way of a spotter, in the presence of a relative humidity of 30% and at room temperature. Chips are thus produced having a density of 100 dots per PAD. An immunochemical analysis like that described in example 4 is carried out on dried blood samples, adsorbed on cellulose plugs pre-treated with a 90% (by volume) glycerol solution in water, stored at room temperature for 15 days, then eluted in 1 ml of Tris-Cl 50 mM, NaCl 150 mM buffer at a pH of 7.2, under continuous stirring for 30 minutes at room temperature. Figure 4B shows the detection of the antigen-antibody complexes (light dots) in every chip. The visible dots having different intensities are quantitatively measured by the densitometric software and the antigens of the different foods that originate the antibody reaction are identified.

### EXAMPLE 6

Solutions are prepared comprising single or mixed antigens and allergens at a concentration of 15 µg/ml of total proteins, extracted and purified from: garlic, apricot, pineapple, peanuts, orange, bananas, basil, beans, broad bean, fennel, strawberry, edible boleti, shrimps, gorgonzola, bread wheat and durum wheat, beet, cocoa, coffee, artichoke, carrot, horse, cauliflower, cucumber, cherry, kiwi, goat milk, caw's milk, sheep milk, lettuce, beer, yeast, lemon, pork, maize, barley, tangerine, almond, butter, aubergine, cod, honey, mozzarella cheese, buffalo mozzarella cheese, hazelnut, gilthead, oregano, Parmesan cheese, cheese, potatoes, pecorino cheese, pepper, black pepper, sweet peppers, peach, chicken, peas, tomato, parsley, rice, ricotta cheese, rosemary, salmon, sole, soya beans, spinach, turkey, tea, trout, eggs, vanilla, veal, clams, pumpkin, zucchini, aspergillus niger, candida albicans, mucor racemosus, penicillium notatum.

The antigens are immobilized on different glass microarrays, each equipped with 16 nitrocellulose PADs, by way of a spotter, in the presence of a relative humidity of 25% and at room temperature. Chips are in this way produced having a density of 100 dots (corresponding to the immobilized antigens) per PAD. Dried blood samples, adsorbed on cotton-buds treated with an 80% (by volume) glycerol solution in water and stored at room temperature for two months, are eluted in 1 ml of Tris-HCl 50 mM, NaCl 150 mM buffer at a pH of 7.2, under continuous stirring for 25 minutes at room temperature and incubated with the above described immobilized antigens.

Figure 4C shows the detection of the antigen-antibody complexes (light dots) in every chip. The visible dots having different intensities are quantitatively measured by using the densitometric software and the antigens of the different foods that give origin to the antibody reaction are identified.

Then, a specifically developed software produces an output wherein the reactivity of the antibodies is expressed as a percentage value, calculated with respect to a 100% value corresponding to the maximum light intensity as measured with the antibodies of the calibration curve present in every PAD.

### EXAMPLE 7

The activity of the immunoglobulins that are present in blood samples dried on non-treated DBS supports, or on DBS supports pre-treated with a 90% (by volume) solution of glycerol in water and stored for 3 months at room temperature, according to a preferred embodiment of the present invention, is compared. The immunological response has been assessed in an immunochemical assay on said samples by way of the microarray technology, as described above, after 2, 7, 15, 21, or 30 days as from samples' adsorption on the supports.

Figure 5 clearly shows that the blood dried on the non-treated DBSs, used for determining the immunological response, results in the formation of haloes on the chip as early as after 2 days. Also, the immunological response is found to be very low.

Conversely, the blood dried on the DBSs pre-treated according to the method of the present invention is found to be biologically active and exhibits an antigenic reactivity as late as after 30 days (Figure 5L). The same result is also achieved with samples analyzed 3 months after their collection on the pre-treated support (not shown). The same results have been achieved with any supports pre-treated according to the invention.

### EXAMPLE 8

In an Enzyme-Linked Immunosorbent Assay (ELISA), the immunological response of immunoglobulins that are present in a sample of plasma separated from fresh blood is assessed, in a dried blood sample on a DBS support pre-treated with a 100% glycerol solution, in accordance with the present invention, stored at room temperature for three months, as well as in a blood sample dried on a non-pre-treated DBS support. Briefly, 24 food and IgG standard antigens at known growing concentrations are immobilized on 96-well micro-titer ELISA plates. After blocking with a buffer comprising 1% BSA for 30 minutes and drying at 30°C for 24 hours, the ELISA plates are washed with a Tris-HCl 50 mM, NaCl 150 mM washing buffer at a pH of 7.2 for 2 minutes. After drying, 100 µl of fresh plasma or dried blood eluted from DBSs pre-treated as described above or dried blood eluted from non-treated DBSs are added to every plate; the samples are diluted from 1 to 600 and incubated for 30 minutes at room temperature with the antigens. After emptying the wells of each individual plate and washing with the washing buffer, a secondary antibody is added and incubated for 30 minutes, then the TMB is added after a further washing. 5 minutes later, diluted sulfuric acid is added to block the reaction, then a read-out is made with a plate reader at 405 nm. The tests made clearly show (Table 1) that the dried blood eluted from non-pre-treated DBSs does not exhibit any reactivity in the ELISA assay, whereas the blood extracted from the DBS pre-treated according to the present invention is found to be active and exhibits an antigenic reactivity comparable to the reactivity exhibited by fresh blood samples, as late as 30 days after laying blood down onto the support. The same results have been achieved with blood samples dried on different supports (small swabs, cotton-buds, supports made from cellulose and their derivatives, or artificial fibers), all pre-treated under the same conditions.

**Table 1. After plate reading at 405 nm, the optical density values are plotted as percentage reactivity value in the range from 0 to 100%, with respect to the maximum optical density value measured on the calibration curve.**

| | Plasma from fresh blood | Blood from DBS pre-treated with 100% glycerol (v/v) | Blood from non-treated DBS |
|---|---|---|---|
| Lamb | 41 | 40 | 4 |
| Oranges | 32 | 31 | 13 |
| Peanuts | 24 | 23 | 9 |
| Cow cheese | 12 | 11 | 1 |
| See bass | 6 | 6 | 0 |
| Chestnut | 7 | 7 | 0 |
| Bean | 12 | 11 | 1 |
| Emmer | 7 | 7 | 0 |
| Melted cheese | 16 | 15 | 3 |
| Strawberry | 7 | 7 | 0 |
| Gorgonzola cheese | 31 | 30 | 8 |
| Bread wheat | 6 | 6 | 0 |
| Pork | 19 | 18 | 8 |
| Apple | 7 | 7 | 0 |
| Mozzarella cheese | 7 | 7 | 0 |
| Peas | 5 | 5 | 0 |
| Chicken | 33 | 32 | 6 |
| Radish | 7 | 6 | 0 |
| Rye | 4 | 4 | 0 |
| Mustard | 32 | 31 | 10 |
| Sole | 25 | 24 | 5 |
| Tea | 12 | 12 | 0 |
| Egg | 23 | 22 | 8 |
| Zucchini | 6 | 6 | 0 |

### EXAMPLE 9

Solutions are prepared comprising antigens, extracted from 92 different foods and purified, at a concentration of 80 µg/ml of total proteins. The foods are the following: anchovies, garlic, apricot, pineapple, duck, peanuts, orange, asparagus, banana, basil, chard, see bass, cocoa, coffee, chamomile, artichoke, carrot, chestnut, horse, cauliflower, chickpeas, cucumber, cherry, onion, rabbit, French beans, emmer, beans, fennel, strawberry, edible boleti, crawfishes, durum wheat, bread wheat, kiwi, goat milk, cow's milk, sheep milk, lettuce, lentils, beer yeast, lemon, pork, maize, barley malt, tangerine, almond, beef meat, apple, aubergine, bilberry, olive, lobster, oregano, barley, Parmesan cheese, potato, black pepper, peppers, pear, peach, pine nuts, peas, chicken, tomato, parsley, plum, rice, rosemary, salmon, celery, cuttlefish, sole, soya bean, spinach, turkey, green tea, tuna fish, trout, eggs, white grapes, vanilla, clams, pumpkin, zucchini.

A glass microarray chip is provided, equipped with 16 nitrocellulose PADs, on which the antigens are immobilized, purified in the presence of 30% of relative humidity and at room temperature, and immobilized on every PAD at a density of 100 dots per PAD, wherein every dot corresponds to one antigen and wherein IgGs at known growing concentrations are immobilized in every PAD. The chip is washed out and blocked as described above.

Blood samples adsorbed on DBSs pre-treated with a 90% (by volume) glycerol solution in water, stored at room temperature, are eluted, one month after depositing them on a pre-treated support, in 1 ml of Tris-HCl 50 mM, NaCl 150 mM buffer at a pH of 7.2, under continuous stirring for 20 minutes at room temperature, then they are incubated with the antigens immobilized on the chip.

After incubation, the immunoglobulins not linked to the antigens are washed away and a human monoclonal anti-IgE secondary antibody provided with peroxidase is interacted with the IgEs linked to the immobilized antigens. After washing, the portion of the peroxidase present in the secondary antibody is reacted with its TMB substrate, to generate visible dots having different intensities (Figure 6). A quantitative measurement of the visible products of the reaction is carried out by using a highly specific dedicated densitometric software, by determining the concentration of the IgEs bound to the antigens, by using, as a calibration curve, the signals provided by the IgEs immobilized on the chip at known growing concentrations.

## Claims

1. A method for immunochemical analyses on dried blood samples, comprising the following sequential steps:
(a) providing a device for collecting a blood sample, comprising a support capable of adsorbing a liquid;
(b) soaking said support with a solution comprising glycerol at a percentage volume concentration (v/v) greater than or equal to about 70% v/v, thus obtaining a pre-treated support;
(c) adsorbing a fresh blood sample onto said pre-treated support and drying said sample adsorbed by the pre-treated support;
(d) analyzing the dried blood sample by way of an immunochemical assay.

2. The method according to claim 1, wherein step (d) of analyzing the dried blood sample by way on an immunochemical assay comprises the following steps:
(d1) eluting the dried blood sample from the pre-treated support;
(d2) incubating one or more antigens with the eluted blood sample, under conditions suitable to allow formation of antigen-antibody complexes;
(d3) detecting antigen-antibody complexes that have formed.

3. The method according to any one of the preceding claims, wherein said support capable of adsorbing a liquid comprises cellulose polymeric fibers or cellulose-derivative fibers or artificial polymeric fibers.

4. The method according to any one of claims 2 and 3, wherein said one or more antigens are immobilized on a solid substrate.

5. The method according to claim 4, wherein said solid substrate is a microarray chip.

6. The method according to any of claims 2 to 5, wherein said immunochemical assay is an ELISA assay.

7. The method according to any one of claims 2 to 6, wherein said one or more antigens are extracted from one or more foods.

8. A device for collecting blood samples, comprising the pre-treated support obtainable at the end of step (b) of the method according to claim 1.

9. A kit for detecting and/or measuring antibodies directed against food antigens in dried blood samples, comprising:
i) the device of claim 8;
ii) a solid substrate on which one or more antigens extracted from one or more foods are immobilized;
iii) a reagent capable of detecting antibodies bound to said one or more antigens.

10. The kit according to claim 9, wherein the solid substrate is a microarray chip.

11. Use of the kit according to claim 10 in a method of *in vitro* diagnosis of food allergies or intolerances in a subject.

12. Use of the device of claim 8 for the collection of blood samples to be dried.

13. A device for collecting blood samples obtainable at the end of step (c) of the method according to claim 1.

14. Use of the device of claim 8 or 13 in a method of immunochemical analysis on a dried blood sample.

15. Use of the device of claim 14, wherein said method of immunochemical analysis is an ELISA assay.

## Patentansprüche

1. Verfahren für immunchemische Analysen an getrockneten Blutproben, umfassend die folgenden aufeinanderfolgenden Schritte:
(a) Bereitstellen einer Vorrichtung zum Entnehmen einer Blutprobe, die einen Träger umfasst, der in der Lage ist, eine Flüssigkeit zu adsorbieren;
(b) Tränken des Trägers mit einer Lösung, umfassend Glycerin in einer prozentualen Volumenkonzentration (Vol./Vol.) größer als oder gleich wie etwa 70 % Vol./Vol., wodurch ein vorbehandelter Träger erlangt wird;
(c) Adsorbieren einer frischen Blutprobe auf dem vorbehandelten Träger und Trocknen der von dem vorbehandelten Träger adsorbierten Probe;
(d) Analysieren der getrockneten Blutprobe mittels eines immunchemischen Tests.

2. Verfahren nach Anspruch 1, wobei Schritt (d) eines Analysierens der getrockneten Blutprobe mittels eines immunchemischen Tests die folgenden Schritte umfasst:
(d1) Eluieren der getrockneten Blutprobe aus dem vorbehandelten Träger;
(d2) Inkubieren eines oder mehrerer Antigene mit der eluierten Blutprobe unter Bedingungen, die geeignet sind, um eine Bildung von Antigen-Antikörper-Komplexen zu ermöglichen;
(d3) Detektieren von Antigen-Antikörper-Komplexen, die sich gebildet haben.

3. Verfahren nach einem der vorherigen Ansprüche, wobei der Träger, der in der Lage ist, eine Flüssigkeit zu adsorbieren, polymere Cellulosefasern oder von Cellulose abgeleitete Fasern oder künstliche Polymerfasern umfasst.

4. Verfahren nach einem der Ansprüche 2 und 3, wobei das eine oder die mehreren Antigene auf einem festen Substrat immobilisiert sind.

5. Verfahren nach Anspruch 4, wobei das feste Substrat ein Mikroarray-Chip ist.

6. Verfahren nach einem der Ansprüche 2 bis 5, wobei der immunchemische Test ein ELISA-Test ist.

7. Verfahren nach einem der Ansprüche 2 bis 6, wobei das eine oder die mehreren Antigene aus einem oder mehreren Nahrungsmitteln extrahiert werden.

8. Vorrichtung zum Entnehmen von Blutproben, umfassend den vorbehandelten Träger, der am Ende von Schritt (b) des Verfahrens nach Anspruch 1 erlangt werden kann.

9. Kit zum Detektieren und/oder zur Messen von Antikörpern, die gegen Nahrungsmittelantigene in getrockneten Blutproben gerichtet sind, umfassend:
i) die Vorrichtung nach Anspruch 8;
ii) ein festes Substrat, auf dem ein oder mehrere Antigene immobilisiert sind, die aus einem oder mehreren Nahrungsmitteln extrahiert wurden;
iii) ein Reagenz, das in der Lage ist, Antikörper zu detektieren, die an dieses eine oder diese mehreren Antigene gebunden sind.

10. Kit nach Anspruch 9, wobei das feste Substrat ein Mikroarray-Chip ist.

11. Verwendung des Kits nach Anspruch 10 in einem Verfahren einer *in-vitro-*Diagnose von Nahrungsmittelallergien oder Nahrungsmittelunverträglichkeiten bei einem Subjekt.

12. Verwendung der Vorrichtung nach Anspruch 8 zum Entnehmen von Blutproben, die getrocknet werden sollen.

13. Vorrichtung zum Entnehmen von Blutproben, die am Ende von Schritt (c) des Verfahrens nach Anspruch 1 erlangt werden können.

14. Verwendung der Vorrichtung nach Anspruch 8 oder 13 in einem immunchemischen Analyseverfahren einer getrockneten Blutprobe.

15. Verwendung der Vorrichtung nach Anspruch 14, wobei das Verfahren der immunchemischen Analyse ein ELISA-Test ist.

## Revendications

1. Méthode pour des analyses immunochimiques sur des échantillons de sang séché, comprenant les étapes séquentielles suivantes :
(a) fourniture d'un dispositif pour la collecte d'un échantillon de sang, comprenant un support capable d'absorber un liquide ;
(b) immersion dudit support dans une solution comprenant du glycérol à une concentration volumique (v/v) en pourcentage supérieure ou égale à environ 70 % v/v, obtenant ainsi un support prétraité ;
(c) absorption d'un échantillon de sang frais sur ledit support prétraité et séchage dudit échantillon absorbé par le support prétraité ;
(d) analyse de l'échantillon de sang séché au moyen d'un dosage immunochimique.

2. Méthode selon la revendication 1, dans laquelle l'étape (d) de l'analyse de l'échantillon de sang séché au moyen d'un dosage immunochimique comprend les étapes suivantes :
(d1) élution de l'échantillon de sang séché à partir du support prétraité ;
(d2) incubation d'un ou plusieurs antigènes avec l'échantillon de sang élué, dans des conditions adaptées à la formation de complexes antigène-anticorps ;
(d3) détection des complexes antigène-anticorps qui ont été formés.

3. Méthode selon l'une quelconque des revendications précédentes, dans laquelle ledit support capable d'absorber un liquide comprend des fibres polymériques cellulosiques, des fibres de dérivé de cellulose ou des fibres polymériques artificielles.

4. Méthode selon l'une quelconque des revendications 2 et 3, dans laquelle un ou plusieurs antigènes sont immobilisés sur un substrat solide.

5. Méthode selon la revendication 4, dans laquelle ledit substrat solide est une puce à ADN.

6. Méthode selon l'une quelconque des revendications 2 à 5, dans laquelle ledit dosage immunochimique est un dosage ELISA.

7. Méthode selon l'une quelconque des revendications 2 à 6, dans laquelle le(s)dit(s) un ou plusieurs antigènes sont extraits d'un ou plusieurs aliments.

8. Dispositif pour collecter des échantillons de sang, comprenant le support prétraité pouvant être obtenu à la fin de l'étape (b) de la méthode selon la revendication 1.

9. Kit pour détecter et/ou mesurer les anticorps dirigés contre les antigènes alimentaires dans des échantillons de sang séché, comprenant :
i) dispositif selon la revendication 8 ;
ii) un substrat solide sur lequel un ou plusieurs antigènes extraits d'un ou plusieurs aliments sont immobilisés ;
iii) un réactif capable de détecter des anticorps liés audit ou auxdits antigènes.

10. Kit selon la revendication 9, dans lequel le substrat solide est une puce à ADN.

11. Utilisation du kit selon la revendication 10 dans une méthode de diagnostic *in vitro* d'allergies ou d'intolérances alimentaires sur un sujet.

12. Utilisation du dispositif selon la revendication 8 pour la collecte d'échantillons de sang à sécher.

13. Dispositif pour collecter des échantillons de sang pouvant être obtenus à la fin de l'étape (c) de la méthode selon la revendication 1.

14. Utilisation du dispositif selon la revendication 8 ou 13 dans une méthode d'analyse immunochimique sur un échantillon de sang séché.

15. Utilisation du dispositif selon la revendication 14, dans laquelle ladite méthode d'analyse immunochimique est un dosage ELISA.
